# EUROPÄISCHE PATENTSCHRIFT

(11) **EP 2 438 960 B1**
(45) Veröffentlichungstag und Bekanntmachung des Hinweises auf die Patenterteilung: **17.06.2015**
(21) Anmeldenummer: 11173713.6
(22) Anmeldetag: 13.07.2011
(51) Int. Cl.: A61N 5/06

(54) **Bestrahlungsvorrichtung mit ergonomischen Anpassungsmöglichkeiten**
Irradiation device with ergonomic adjustment options
Dispositif de rayonnement avec capacités de mise à dimension ergonomiques

(30) Priorität: 06.10.2010 DE 102010047494
(43) Veröffentlichungstag der Anmeldung: 11.04.2012
(73) Patentinhaber: Herbert Waldmann GmbH & Co. KG, 78056 Villingen-Schwenningen (DE)
(72) Erfinder: Heinzler, Marcus, 78595 Hausen o.V. (DE)
(74) Vertreter: Westphal, Mussgnug & Partner Patentanwälte mbB

(56) Entgegenhaltungen:
- WO-A1-02/098508
- WO-A1-2006/048162
- WO-A1-2009/133385
- WO-A2-2005/030317
- DE-A1- 3 602 940
- DE-U1- 20 317 167
- DE-U1-202009 013 594
- GB-A- 2 082 747

## Beschreibung

Die vorliegende Erfindung betrifft eine Bestrahlungsvorrichtung zum Bestrahlen von Körperteilen eines Patienten mit einem Trägersystem, mindestens einem Leuchtenkopf und einem Bedienelement.

Aus dem Stand der Technik, wie z.B. DE 203 17 167 U1, WO 2005/030317 oder DE 36 02 940, sind Bestrahlungsvorrichtungen zum Bestrahlen von Körperteilen eines Patienten als sogenannte Teilkörper-Therapiegeräte beispielsweise zum Bestrahlen von Händen, Füßen oder der Brust, dem Rücken, Steiß, Knie oder Unterschenkel im Rahmen einer sogenannten UV-Phototherapie bekannt. Die bekannten Therapiegeräte bestehen in der Regel aus einem Geräteträger, der als Rollwagen ausgeführt ist. An dem Geräteträger sind Leuchtenköpfe, sogenannte Behandlungsköpfe, befestigt, die jeweils eine Lichtquelle sowie Vorschaltgeräte zu deren Steuerung umfassen. Infolgedessen weisen die Behandlungsköpfe ein hohes Gewicht auf und sind daher in der Regel fest mit dem Geräteträger verschraubt oder statisch verrastet. Eine Ausrichtung der Behandlungsköpfe erfolgt in der Regel einmalig bei der Montage des Gerätes unter Verwendung von speziellem Werkzeug. Daher ist es bei diesen Geräten nicht möglich, die Behandlungsköpfe beziehungsweise das gesamte Gerät individuell auf einen einzelnen zu therapierenden Patienten einzustellen, um somit eine ergonomische Anpassung durchzuführen. Vielmehr hat sich der Patient an die Bestrahlungsvorrichtung anzupassen und muss dementsprechend relativ zu dem Gerät positioniert werden. Dies bedeutet gegebenenfalls, dass der Patient über die gesamte Behandlungsdauer, die unter Umständen mehrere Minuten dauern kann, in einer unbequemen Position verharren muss.

Auf Grund der fest verbauten Behandlungsköpfe kann das Therapiegerät darüber hinaus nicht an verschiedene Behandlungsformen angepasst werden, da es nicht möglich ist, die Behandlungsköpfe entsprechend auszurichten. Während eine Abstrahlfläche zur Bestrahlung von Händen möglichst waagerecht auszurichten ist, sollte eine Abstrahlfläche für eine Gesichtsbehandlung im Wesentlichen einen steilen Anstellwinkel aufweisen, um parallel zu dem Gesicht zu stehen. Eine Änderung der Ausrichtung der Behandlungsköpfe ist nur unter Verwendung von entsprechendem Werkzeug möglich und setzt eine ausreichende Schwenkbarkeit der Behandlungsköpfe voraus. Es kann daher aufgrund des erforderlichen Aufwandes nicht individuell für den jeweiligen Patienten angepasst werden.

Ein weiterer Nachteil ergibt sich aufgrund der jeweils in den einzelnen Behandlungsköpfen integrierten Steuerungen und Bedienelementen sowie deren hierdurch bedingten dezentralen Anordnung, wodurch eine Bedienung des gesamten Therapiegerätes umständlich wird. Dies hat zur Folge, dass der Anwender zur Bedienung direkten Zugang zu dem jeweiligen Behandlungskopf benötigt. Insbesondere Behandlungsköpfe, die in einem unteren Bereich des Therapiegerätes angeordnet sind, erfordern eine gebückte Haltung zur Bedienung durch den Anwender. Weisen die Therapiegeräte eine gewisse Höhe auf und sind in diesem Bereich Behandlungsköpfe vorgesehen, so bereiten deren Bedienelemente insbesondere kleinen Menschen erhebliche Schwierigkeiten bei der Bedienung und beim Ablesen von entsprechenden Anzeigen. Des Weiteren ist es bei den aus dem Stand der Technik bekannten Therapiegeräten erforderlich, dass der Anwender zur Bedienung des Gerätes vor diesem steht, um die an den Behandlungsköpfen angeordneten Bedienelemente zu betätigen. Es ist daher nicht möglich, den Patienten bereits vor der Bedienung des Gerätes vor diesem zu platzieren. Vielmehr müssen jegliche Einstellungen bereits im Voraus vollständig abgeschlossen sein.

Je nach Therapie kann es erforderlich sein, die verwendeten Behandlungsköpfe durch Behandlungsköpfe mit einer anderen Lichtquelle, wie beispielsweise UV-A und UV-B Lichtquellen, zu ersetzen. Dies erfordert bei den bekannten Geräten, wie voranstehend beschrieben, den Einsatz von zusätzlichem Werkzeug. Darüber hinaus ist ein derartiger Austausch aufgrund des bereits erwähnten erheblichen Gewichts der Behandlungsköpfe nur mit erhöhtem Kraftaufwand möglich.

Da jeder Behandlungskopf sein eigenes Steuerelement und ein Bedienelement aufweist, ist darüber hinaus nicht nur die Bedienung der einzelnen Bedienelemente umständlich, vielmehr ist auch eine entsprechend hohe Anzahl von einzelnen Steuerelementen beziehungsweise Bedienelemente notwendig, wodurch ein hoher Ressourcen- und Kostenaufwand begründet wird.

Aufgabe der Erfindung ist es daher, eine Bestrahlungsvorrichtung bereitzustellen, die individuell und ergonomisch an den zu behandelnden Patienten anpassbar ist, sodass dieser bequem vor dem Gerät platziert werden kann. Darüber hinaus soll die Bestrahlungsvorrichtung an die verschiedenen Behandlungsformen und Therapien anpassbar sowie einfach und komfortabel zu bedienen sein.

Die Aufgabe wird erfindungsgemäß mit einer Bestrahlungsvorrichtung gemäß Anspruch 1 gelöst. Vorteilhafte Ausgestaltungen ergeben sich aus den Unteransprüchen.

Demnach wird eine Bestrahlungsvorrichtung zum Bestrahlen von Körperteilen eines Patienten bereitgestellt, die ein Trägersystem umfasst, welches ein Grundgerüst der Bestrahlungsvorrichtung bereitstellt. Des Weiteren umfasst die Bestrahlungsvorrichtung mehrere Leuchtenköpfe mit Lichtquellen für das Bestrahlen und ein Bedienelement zum Bedienen der Bestrahlungsvorrichtung. Die Bestrahlungsvorrichtung umfasst außerdem ein mit dem Trägersystem verbundenes, separates Steuerungsmodul mit mindestens einer Steuerung zum Steuern des mindestens einen Leuchtenkopfes.

Demzufolge ist die Steuerung mehrerer Leuchtenköpfe als eine gemeinsame, separate Vorrichtung ausgebildet und nicht in den jeweiligen Leuchtenköpfen integriert. Eine derartige Steuerung umfasst unter anderem Vorschaltgeräte, insbesondere magnetische Vorschaltgeräte, und weitere elektronische Bauteile zum Steuern der Lichtquellen in dem mindestens einen Leuchtenkopf. Dies bietet die Möglichkeit, das Gewicht des jeweiligen Leuchtenkopfes erheblich zu reduzieren und die genannten Bauteile in das eigenständige, separate Steuerungsmodul zu integrieren und dort zu kombinieren. Es kann somit eine einzige und gemeinsame Steuerung mehrerer Leuchtenköpfe bereitgestellt werden. Vorzugsweise können aufgrund der Kombination einzelne Bauteile und Steuerungen gemeinsam genutzt und somit Ressourcen eingespart werden.

Das separate Steuerungsmodul ist als Schaltschrank oder Rack ausgebildet. Unter einem Schaltschrank ist insbesondere ein Gehäuse zu verstehen, das die der Steuerung zum Steuern der Leuchtenköpfe zugeordneten Komponenten und elektronischen Bauteile umfasst. Beispielsweise weist der Schaltschrank eine im Wesentlichen langgestreckte quaderförmige Bauform auf. Vorzugsweise ist der Schaltschrank plattenförmig mit einer bezüglich seiner Breite geringen Tiefe und großen Höhe ausgebildet. Ebenfalls vorzugsweise weist der Schaltschrank mindestens eine ebene, im Betriebszustand dem Patienten zugewandte Vorderseite auf.

Die Leuchtenköpfe sind modular ausgeführt und lösbar mit dem Steuerungsmodul verbunden. Dies bedeutet, dass der Leuchtenkopf ausschließlich an dem Steuerungsmodul angeordnet ist, jedoch keine eigene Verbindung zu dem Trägersystem aufweist. Des Weiteren ist der Leuchtenkopf aufgrund seiner modularen Bauweise und der lösbaren Verbindung auswechselbar an dem Steuerungsmodul angeordnet und kann bei Bedarf entfernt oder ersetzt werden. Die lösbare Verbindung ist hierzu vorzugsweise derart ausgestaltet, dass der Leuchtenkopf ohne einen Einsatz von Werkzeug entfernbar beziehungsweise einsetzbar, jedoch für einen sicheren Betrieb ausreichend fixiert ist.

Das Steuerungsmodul ist relativ zu dem Trägersystem um eine erste Schwenkachse schwenkbar mit dem Trägersystem verbunden. Auf diese Weise lässt sich das Steuerungsmodul bezüglich des Trägersystems verschwenken, um die Bestrahlungsvorrichtung an den zu therapierenden Patienten individuell anzupassen. Beispielsweise kann das Trägersystem einen im Wesentlichen U-förmigen Aufbau aufweisen, wobei an den beiden äußeren Enden der beiden Schenkel des u-förmigen Trägersystems die Schwenkachse gelagert ist, sodass diese die beiden Enden miteinander verbindet.

Die Leuchtenkopfe sind relativ zu dem Steuerungsmodul um jeweils eine zweite Schwenkachse schwenkbar. Dies ermöglicht eine verbesserte, universelle Einsetzbarkeit des jeweiligen Leuchtenkopfes, da dieser je nach Anwendung in die hierfür optimale Position geschwenkt werden kann. Soll beispielsweise ein Handrücken eines vor dem Gerät platzierten Patienten bestrahlt werden, so kann der Leuchtenkopf derart geschwenkt werden, dass seine Abstrahlrichtung beispielsweise parallel zu der Hand ausgerichtet wird. Soll dagegen das Gesicht des Patienten bestrahlt werden, so kann die Abstrahlrichtung durch Schwenken des Leuchtenkopfes in die gewünschte Richtung gedreht werden.

Vorzugsweise sind die erste Schwenkachse und die jeweils zweite Schwenkachse im Wesentlichen parallel zueinander ausgerichtet. Dies ermöglicht eine besonders vorteilhafte und flexible Anpassung der Bestrahlungsvorrichtung an eine unterschiedliche Körpergröße der zu therapierenden Patienten. Beispielsweise können sowohl die erste als auch die zweite Schwenkachse im Betriebszustand im Wesentlichen horizontal ausgerichtet sein.

Gemäß einer weiteren Ausführungsform ist der mindestens eine Leuchtenkopf kommunikationstechnisch zum Austausch von Informationen mit dem separaten Steuerungsmodul verbunden. Ein Austausch von Informationen zwischen dem Leuchtenkopf und dem Steuerungsmodul ist insbesondere zur Berücksichtigung der verwendeten Lampenart von Bedeutung, um eine Fehlfunktion oder fehlerhafte Strahlungsdossierung auszuschließen und die Sicherheit für den Patienten zu gewährleisten.

Aufgrund der Möglichkeit der Austauschbarkeit des mindestens einen Leuchtenkopfes ist das Steuerungsmodul vorzugsweise dazu ausgebildet, den jeweiligen Leuchtenkopf zu identifizieren. Hierzu gehört eine Erkennung eines verwendeten Steckplatzes, an dem der jeweilige Leuchtenkopf angeordnet ist sowie die Fähigkeit, ein in dem Leuchtenkopf vorgesehenes Speichermedium auszulesen. In diesem Speichermedium sind beispielsweise mindestens eine der folgenden Spezifikationen hinterlegt: Leuchtmittelbestückung, d. h. Art der Lichtquelle, Intensität, Betriebsdauer und/oder Seriennummer des jeweiligen Leuchtkopfes. Selbstverständlich können ebenso andere oder zusätzliche Spezifikationen hinterlegt sein.

Ausgelesen werden diese Spezifikationen vorzugsweise mittels eines Bus-Systems zu vordefinierten Zeitpunkten, in regelmäßigen Abständen oder nach dem Andocken eines neuen Leuchtenkopfes an das Steuerungsmodul.

Gemäß einer bevorzugten Ausführungsform verläuft die erste Schwenkachse im Wesentlichen durch einen Schwerpunkt des Steuerungsmodules. Somit können Drehmomente aufgrund eines Eigengewichts des Steuerungsmodules reduziert und eine Standsicherheit der Bestrahlungsvorrichtung erhöht werden.

Gemäß einer anderen Ausführungsform kann das Trägersystem fahrbar zum Verfahren der Bestrahlungsvorrichtung ausgebildet sein. Dies ermöglicht eine besonders einfache Handhabung und universelle Einsetzbarkeit der Bestrahlungsvorrichtung.

Vorzugsweise ist der mindestens eine Leuchtenkopf jeweils mittels lösbarer Steckverbindungen mit dem Steuerungsmodul verbunden.

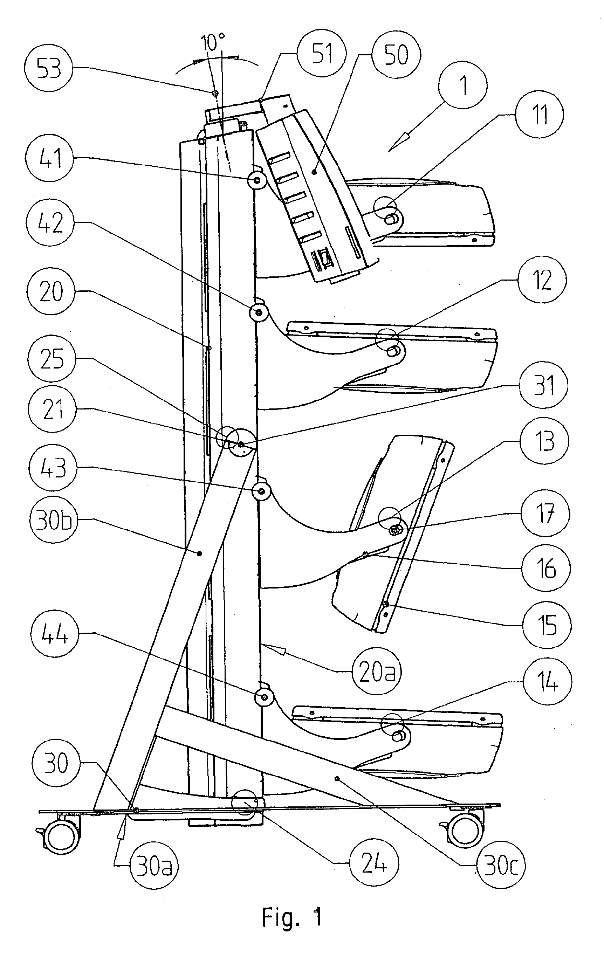

Hierzu weist das Steuerungsmodul entsprechende Aufnahmen in Form von Steckplätzen auf, in die der mindestens eine Leuchtenkopf gesteckt werden kann. Sind mehrere dieser Steckplätze vorhanden, so ist der eine oder die mehreren Leuchtenköpfe in beliebiger Kombination auf diesen platzierbar. Vorzugsweise können die lösbaren Steckverbindungen mit weiteren form- und/oder kraftschlüssigen Verbindungsformen kombiniert werden. Vorzugsweise kann eine kombinierte Verbindung zum Einhängen und anschließendem Einrasten mittels Verschwenken des Leuchtenkopfes gemäß den Figuren 1 bis 12 vorgesehen sein.

Gemäß einer weiteren Ausführungsform ist das Bedienelement der Bestrahlungsvorrichtung schwenkbar um mindestens eine dritte Schwenkachse mit dem Steuerungsmodul verbunden. Das Bedienelement kann somit in verschiedene Stellungen bewegt werden und ermöglicht so eine individuelle Anpassung an den jeweiligen Einsatzzweck oder an das Bedienpersonal. Vorzugsweise ist das Bedienelement über einen Schwenkarm schwenkbar mit dem Steuerungsmodul verbunden und kann in definierte Schwenkpositionen einrasten, sodass ein versehentliches Verschwenken während der Bedienung des Bedienelementes verhindert wird. Die entsprechende Verrastung kann derart ausgestaltet sein, dass beispielsweise zum Verstellen das Bedienelement oder der Schwenkarm zunächst aus einer verrasteten Stellung angehoben werden muss, um das Bedienelement beziehungsweise den Schwenkarm in die gewünschte Position zu schwenken. Vorzugsweise rastet das Bedienelement beziehungsweise der Schwenkarm in der gewünschten Stellung wieder selbstständig ein.

Gemäß einer bevorzugten Ausführungsform ist der mindestens eine Leuchtenkopf um einen Winkel schwenkbar, der sich aus dem Betrag eines Schwenkwinkels des Steuerungsmoduls um die erste Schwenkachse und weiteren 180° zusammensetzt. Kann also das Schwenkelement um bis zu 20° geschwenkt werden, so ist der Leuchtenkopf um mindestens 200° um dessen zweite Schwenkachse schwenkbar. Dies bietet die Möglichkeit, den Leuchtenkopf sowohl zur Abstrahlung in einer ersten Richtung als auch in einer um 180° entgegengesetzten Richtung hierzu zu betreiben, selbst wenn zusätzlich das Steuerungsmodul verschwenkt ist. Zeigt die Abstrahlrichtung des Leuchtenkopfes beispielsweise in einer ersten Stellung zum Bestrahlen eines Fußes senkrecht nach unten, so kann der Leuchtenkopf über den genannten Winkelbereich hinweg derart verschwenkt werden, dass die Abstrahlrichtung beispielsweise in entgegengesetzter Richtung nach oben zeigt.

Beispielsweise kann der mindestens eine Leuchtenkopf jeweils mindestens einen Tragarm zum lösbaren Verbinden mit mindestens einem an dem Steuerungsmodul ausgebildeten Aufnahmeelement zur mindestens teilweisen Aufnahme des mindestens einen Tragarms umfassen. Beispielsweise sind die Aufnahmeelemente, im Folgenden auch als Aufnahmen bezeichnet, an der beschriebenen, dem Patienten im Betrieb zugewandten Vorderseite angeordnet.

Vorzugsweise weist der Tragarm eine U-Form auf, wobei ein Gehäuse des Leuchtenkopfes zwischen den beiden Schenkeln des U-förmigen Tragarms schwenkbar zu diesem angeordnet ist. Ein die beiden Schenkel verbindender Grundabschnitt des U-förmigen Tragarms ist in einem montierten Zustand mit dem Steuerungsmodul verbunden.

Vorzugsweise ist der Grundabschnitt des U-förmigen Tragarmes im montierten Zustand mit der dem Patienten im Betrieb zugewandten Vorderseite verbunden.

Des Weiteren kann der mindestens eine Tragarm mindestens einen Rasthebel zum wahlweisen Verrasten des Leuchtenkopfes an dem Steuerungsmodul umfassen. Hierzu weist das Steuerungsmodul entsprechend ausgebildete Aufnahmeelemente auf, in die der Rasthebel mindestens abschnittsweise mittels einer Rastnase eingreifen kann.

Darüber hinaus kann der mindestens eine Leuchtenkopf mittels einer Rastanordnung relativ zu dem Steuerungsmodul wahlweise fixierbar sein. Mittels der Rastanordnung kann somit die Schwenkbewegung des Leuchtenkopfes um die zweite Schwenkachse wahlweise arretiert und der Leuchtenkopf entsprechend ausgerichtet werden.

Des weiteren wird erfindungsgemäß ein Leuchtenkopf vorgeschlagen, der gemäß der gegebenen Beschreibung ausgebildet ist. Ebenso wird ein gemäß der gegebenen Beschreibung ausgebildetes Rack bestehend aus mindestens dem Trägersystem und einem Bedienelement sowie einem gemäß der Beschreibung ausgebildeten Steuerungsmodul zur Aufnahme eines entsprechend ausgebildeten Leuchtenkopfes vorgeschlagen.

Der Gegenstand der Erfindung ist anhand der in den nachfolgenden Figuren dargestellten Ausführungsformen näher erläutert. Hierbei zeigt
- Fig. 1: eine erfindungsgemäße Bestrahlungsvorrichtung in vertikaler Stellung in einer Seitenansicht,
- Fig. 2: die erfindungsgemäße Bestrahlungsvorrichtung gemäß Figur 1 in verschwenkter Stellung,
- Fig. 3: die erfindungsgemäße Bestrahlungsvorrichtung aus Figur 1 in einer Draufsicht mit ausgeklapptem Bedienelement,
- Fig. 4: die erfindungsgemäße Bestrahlungsvorrichtung aus Figur 1 in einer weiteren Draufsicht mit angeklapptem Bedienelement,
- Fig. 5: einen erfindungsgemäßen Leuchtenkopf für eine Bestrahlungsvorrichtung nach den Figuren 1-4 in einer Seitenansicht in zwei verschwenkten Stellungen,
- Fig. 6: die erfindungsgemäße Bestrahlungsvorrichtung in einer Vorderansicht mit einem eingebauten Leuchtenkopf,
- Fig. 7: eine seitliche Ansicht eines Leuchtenkopfes in eingebautem Zustand, mit einer seitlichen Detailansicht einer Rastanordnung sowie einer seitlichen Detailansicht eines Rasthebels,
- Fig. 8: eine Draufsicht der erfindungsgemäßen Bestrahlungsvorrichtung mit einer geschnittenen Detailansicht der Rastanordnung sowie einer geschnittenen Detailansicht einer Kontaktverbindung eines Leuchtenkopfes mit der Bestrahlungsvorrichtung,
- Fig. 9: eine weitere seitliche Ansicht des Leuchtenkopfes,
- Fig. 10: eine weitere seitliche Ansicht des Leuchtenkopfes gemäß Figur 7 mit gelöster Rastanordnung,
- Fig. 11+12: den Leuchtenkopf gemäß den Figuren 9 und 10 in einem entriegelten Zustand des Rasthebels und der Rastanordnung.

Figur 1 zeigt eine erfindungsgemäße Bestrahlungsvorrichtung 1 in vertikaler Stellung. Die Bestrahlungsvorrichtung 1 umfasst ein Trägersystem 30, das ein Grundgerüst der Bestrahlungsvorrichtung 1 bereitstellt und dessen weitere Komponenten trägt.

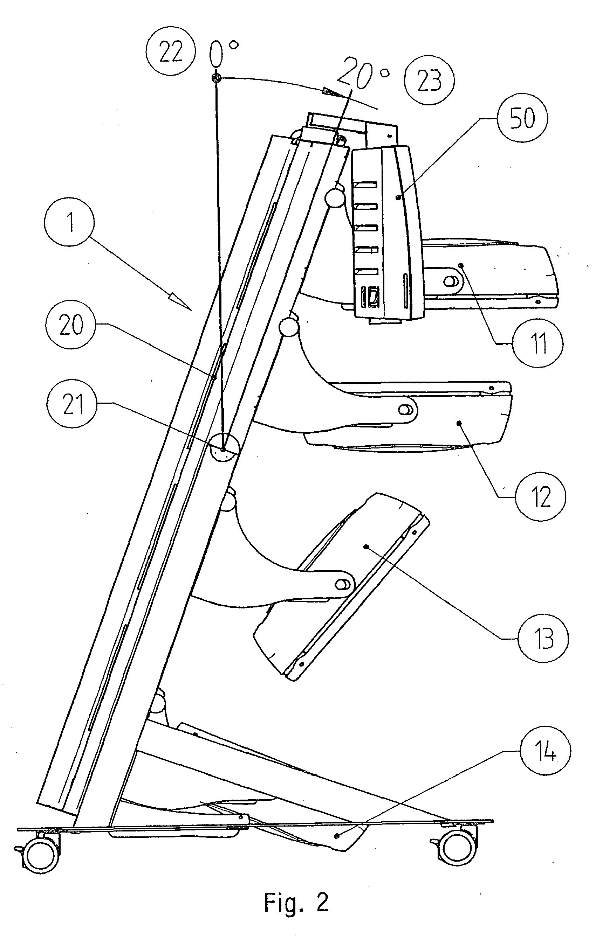

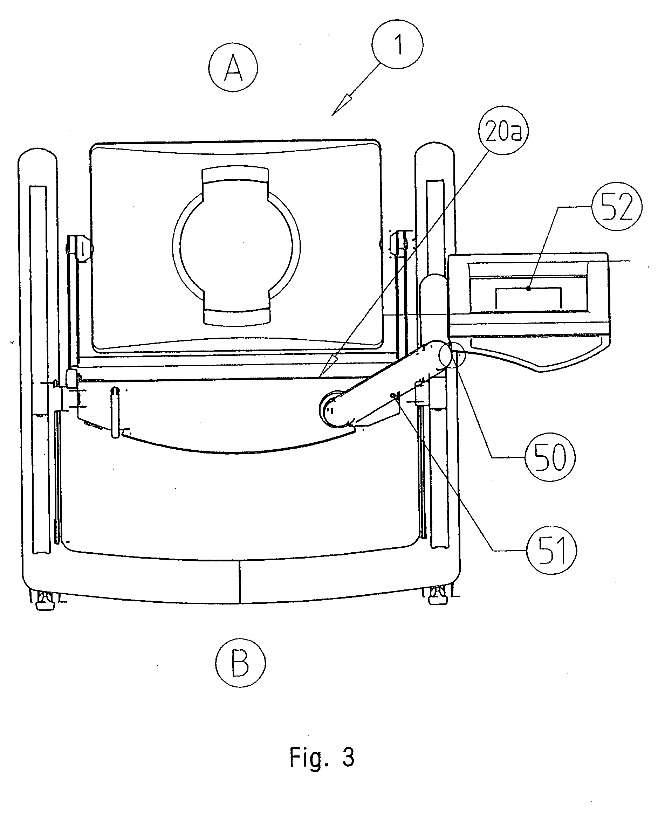

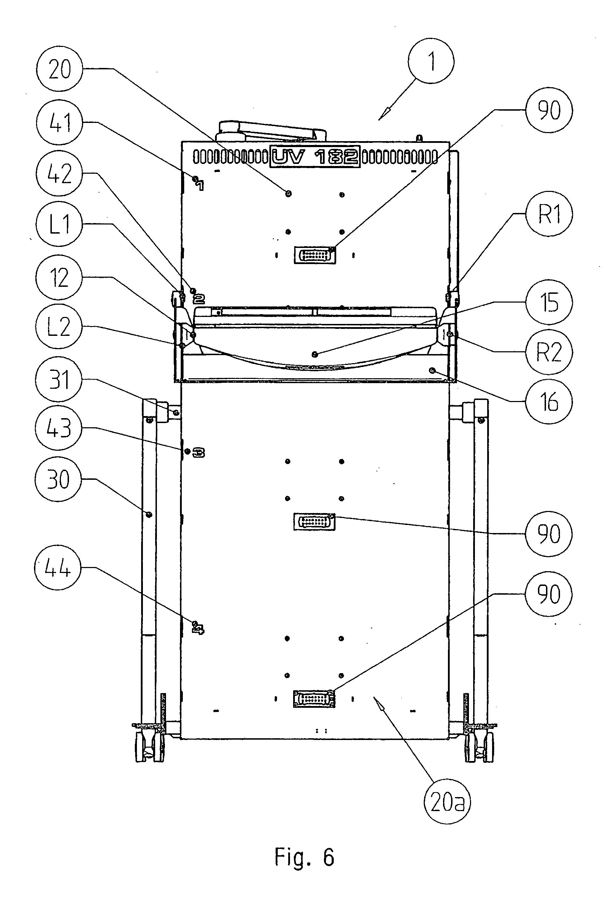

Es ist in der dargestellten Ausführungsform mittels Rollen an einem Fuß 30a des Trägersystems 30 verfahrbar ausgebildet. Das Trägersystem 30 weist zwei von dem Fuß 30a in im Wesentlichen vertikaler Richtung erstreckte Schenkel 30b auf, die jeweils mittels einer Strebe 30c an dem Fuß 30a abgestützt sind. Die Schenkel 30b bilden zusammen mit dem Fuß 30a eine im Wesentlichen U-förmige Bauform des Trägersystems 30 (vgl. Figuren 3 und 6). Jeder der beiden Schenkel 30b weist an einem dem Fuß 30a abgewandten Ende ein Lager 21 auf, in welchem eine erste Schwenkachse 31 gelagert ist. Diese verbindet schwenkbar zu dem Trägersystem 30 ein separates Steuerungsmodul 20, in welchem Steuerungen zum Steuern von Leuchtenköpfen 11, 12, 13, 14 der Bestrahlungsvorrichtung 1, insbesondere deren Vorschaltgeräte, angeordnet sind. Somit kann das Gewicht der einzelnen Leuchtenköpfe 11-14 reduziert werden. Das dargestellte Steuerungsmodul 20 ist als Schaltschrank ausgebildet, der mit einer Längserstreckung in vertikaler Richtung ausgerichtet und im Wesentlichen plattenförmig mit einer gewölbten Rückseite ausgebildet ist. In der dargestellten Ausführungsform ist das Steuerungsmodul 20 in seinem Schwerpunkt mittels der ersten Schwenkachse 31 an dem Trägersystem 30 aufgehängt und lässt sich um die erste Schwenkachse 31 schwenken. Die Schwenkbewegung kann beispielsweise in den Lagern 21 durch Anschläge (nicht dargestellt) beschränkt sein. Vorzugsweise ist eine Schwenkbewegung des Steuerungsmoduls 20 aus der in Figur 1 dargestellten Ausgangslage mit einem Neigungswinkel von 0° gegenüber der Senkrechten in eine verschwenkte Stellung gemäß Figur 2 mit einem Neigungswinkel von beispielsweise 20° gegenüber der Senkrechten beziehungsweise der Ausgangslage für eine anatomische und ergonomische Anpassung an einen zu bestrahlenden Patienten möglich. Das Verschwenken des Steuerungsmoduls 20 kann mittels eines Getriebes 24 oder 25 vorgenommen werden.

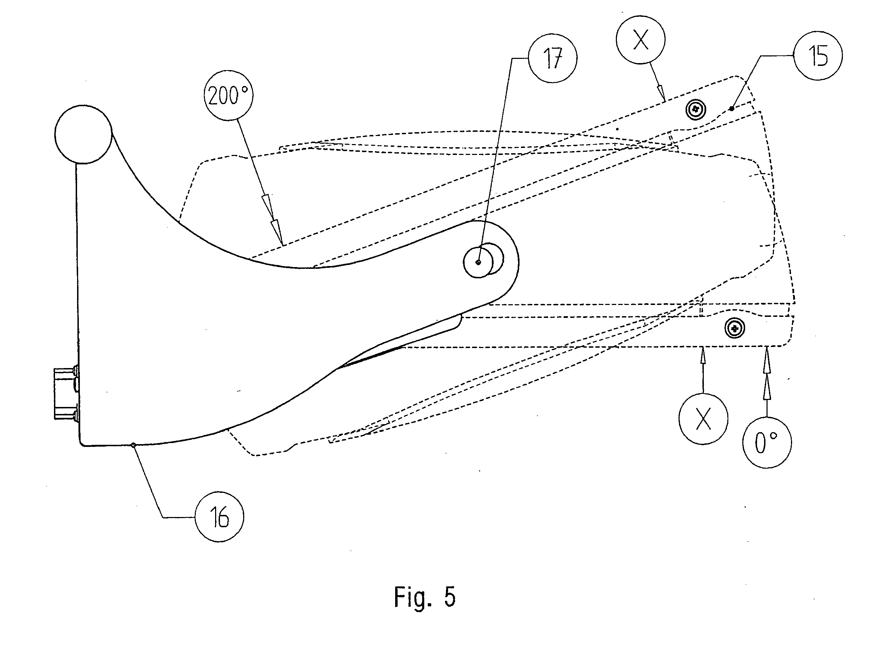

Das beschriebene, als Schaltschrank ausgebildete Steuerungsmodul 20 weist eine erste Vorderseite 20a auf, die in einem Betriebszustand einem zu bestrahlenden Patienten zugewandt ist. An dieser Vorderseite 20a sind mehrere Aufnahmen 41, 42, 43, 44 zum lösbaren Verbinden der modular ausgestalteten Leuchtenköpfe 11-14 mit dem Steuerungsmodul 20 angeordnet. Die dargestellten Leuchtenköpfe 11-14 weisen Tragarme 16 auf, welche an einem dem jeweiligen Leuchtenkopf abgewandten Ende Schwenkbolzen zum Einhängen in die Aufnahmen 41-44 umfassen. Nach dem Einhängen der Schwenkbolzen in die Aufnahmen 41-44 des Steuerungsmoduls 20 kann der jeweilige Leuchtenkopf in einer Abwärtsbewegung geschwenkt und mit einer unterhalb des Schwenkbolzens angeordneten Steckverbindung mit dem Steuerungsmodul 20 verbunden beziehungsweise lösbar in dieses eingerastet werden (vgl. Figur 5). In der dargestellten Ausführungsform sind vier Aufnahmen 41-44 für Leuchtenköpfe 11-14 dargestellt, die beliebig belegt und kombiniert werden können. Beispielsweise können neben der dargestellten Belegung aller vier Aufnahmen 41-44 ebenso lediglich eine, zwei oder drei der Aufnahmen 41-44 in beliebiger Kombination belegt werden, wodurch eine besonders flexible Möglichkeit zur Kombination der Leuchtenköpfe 11-14 erzielt wird. Eine dabei entstehende unterschiedliche Schwerpunktslage des schwenkbaren Steuerungsmoduls 20 kann durch eine Selbsthemmung des Getriebes 24 oder 25 abgefangen werden. Selbstverständlich ist es ebenso möglich, eine entsprechende Bestrahlungsvorrichtung 1 mit einer größeren oder geringeren Anzahl von Aufnahmen 41 - 44 und Leuchtenköpfen 11-14 vorzusehen.

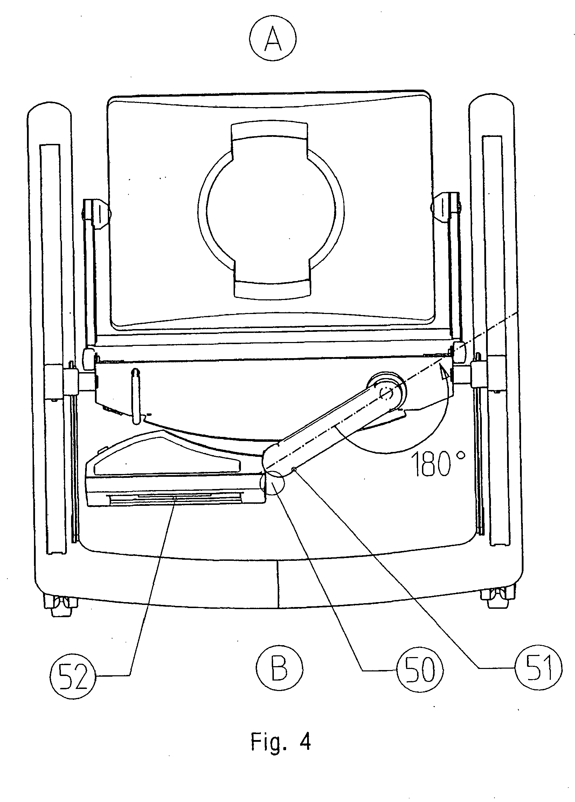

Jeder der Leuchtenköpfe 11-14 umfasst ein Gehäuse 15, das um eine zweite Schwenkachse 17 gegenüber dem jeweiligen Tragarm 16 beziehungsweise dem Steuerungsmodul 20 schwenkbar ist, wodurch die Leuchtenköpfe 11-14 universell zur Bestrahlung unterschiedlichster Körperpartien individuell angepasst werden können. Darüber hinaus weist die Bestrahlungsvorrichtung 1 ein Bedienelement 50 zum Bedienen der Bestrahlungsvorrichtung 1 auf, insbesondere zum zentralen Steuern der Leuchtenköpfe 11-14 über das gemeinsame Bedienelement 50. Dieses ist um eine dritte Schwenkachse 53 gegenüber dem Steuerungsmodul 20 schwenkbar. Das Bedienelement 50 kann somit in eine Position im Bereich der Vorderseite 20a des Steuerungsmoduls 20 gemäß den Figuren 1-3 geschwenkt und bedient werden. Alternativ kann das Bedienelement 50 gemäß Figur 4 auf eine Rückseite der Bestrahlungsvorrichtung 1 verschwenkt werden. Hierzu ist das Bedienelement 50 der Bestrahlungsvorrichtung 1 um mindestens 180° schwenkbar. Vorzugsweise ist das Bedienelement 50 mittels eines Schwenkarmes 51 mit dem Steuerungsmodul 20 verbunden, der in seinen Endstellungen einrastbar ausgebildet ist, sodass ein versehentliches Verdrehen während einer Bedienung des Bedienelements 50 verhindert wird. Zum Verstellen des Bedienelements 50 beziehungsweise des Schwenkarms 51 muss dieser aus der Verrastung angehoben werden und kann anschließend in eine gewünschte Position gebracht werden. Eine theoretisch entstehende Negativneigung des Bedienelementes 50 im Falle eines verschwenkten Steuerungsmodules 20 gemäß Figur 2 wird durch eine geneigte Anordnung der dritten Schwenkachse 53 sowie eine entsprechende Konstruktion des Schwenkarmes 51 begegnet. Das Bedienelement 50 steht somit auch bei geneigtem Steuerungsmodul 20 zumindest senkrecht. So kann auf weitere Gelenke verzichtet werden und die Bestrahlungsvorrichtung 1 optimal bedient werden.

Figur 2 zeigt, wie bereits erwähnt, die erfindungsgemäße Bestrahlungsvorrichtung 1 in verschwenkter Stellung 23. Aufgrund der Neigung des Steuerungsmodules 20 um einen Neigungswinkel von 20° gegenüber der Senkrechten 22 bewegen sich die oberhalb des Lagers 21 angeordneten Leuchtenköpfe 11 und 12 auf einen vor der Bestrahlungsvorrichtung 1 sitzenden Patienten zu. Die unterhalb des Lagers 21 angeordneten Leuchtenköpfe 13 und 14 werden dagegen von dem Patienten weggeschwenkt. Auf diese Weise kann die beschriebene ergonomische Anpassung an den jeweiligen Patienten erfolgen. Beispielsweise kann bei großen Patienten das Steuerungsmodul 20 in die verschwenkte Position 23 gebracht werden, wohingegen bei kleinen Patienten das Steuerungsmodul 20 in die aufrechte Stellung 22 gebracht wird, da sich in der aufrechten Stellung 22 der Abstand der Leuchtenköpfe 13 und 14 zur Bestrahlung von Füßen zu den Leuchtenköpfen 11 und 12 zur Bestrahlung von Händen reduziert.

Figur 3 zeigt die erfindungsgemäße Bestrahlungsvorrichtung 1 aus Figur 1 in einer Draufsicht mit ausgeklapptem Bedienelement 50, dessen Bedienfeld 52 in die gleiche Richtung A weist wie die Vorderseite 20a.

In Figur 4 ist die erfindungsgemäße Bestrahlungsvorrichtung 1 gemäß den Figuren 1 und 3 dargestellt, wobei das Bedienelement 50 um 180° gegenüber der in Figur 3 dargestellten Position verschwenkt und somit auf einer Rückseite B der Bestrahlungsvorrichtung 1 angeordnet ist. Das Bedienfeld 52 ist somit von der Rückseite der Bestrahlungsvorrichtung 1 zu bedienen.

Figur 5 zeigt einen erfindungsgemäßen Leuchtenkopf 12 für eine Bestrahlungsvorrichtung 1 nach einer der Figuren 1-4 in einer Seitenansicht in zwei unterschiedlichen verschwenkten Stellungen 0° und 200°, die jeweils gestrichelt dargestellt sind. Als Referenzfläche dient jeweils eine Abstrahlfläche beziehungsweise eine Lichtaustrittsfläche X des Gehäuses 15, die in einer Ausgangsstellung 0° mit Schwenkwinkel 0° senkrecht nach unten gerichtet ist und in einer um 200° geschwenkten Position schräg nach oben weist. Das Gehäuse 15 des Leuchtenkopfes 12 kann hierzu gegenüber seinem Tragarm 16 um die zweite Schwenkachse 17 geschwenkt werden. So ist es möglich, die Lichtaustrittsfläche X auch im Falle des verschwenkten Steuerungsmoduls 20 waagerecht nach oben beziehungsweise waagerecht nach unten auszurichten. Dies gewährleistet eine optimale Anpassung an die Ergonomie des Patienten und eine gewählte Behandlungsform. Vorzugsweise werden Anschlusskabel für die in dem Gehäuse 15 angeordneten Leuchtmittel des Leuchtenkopfes durch einen Mittelpunkt der zweiten Schwenkachse 17 verlegt sowie ebenfalls vorzugsweise verdeckt in dem Tragarm 16 bis zu dessen abgewandtem Ende zum Verbinden mit dem Steuerungsmodul geführt (Verkabelung nicht dargestellt). Auf diese Weise wird die beschriebene Schwenkbarkeit des Gehäuses 15 nicht durch störende Kabel beeinträchtigt. Der dargestellte Leuchtenkopf 12 weist auf seiner Oberseite eine gebogene Form auf, sodass sich eine zu den Seitenflächen des Gehäuses 15 schräg abfallende Kontur des Gehäuses 15 ergibt, die es ermöglicht, die Abstände der Leuchtenköpfe in verbautem Zustand auf ein Minimum zu reduzieren, sodass sich diese nicht gegenseitig beim Schwenken beeinflussen.

Figur 6 zeigt die erfindungsgemäße Bestrahlungsvorrichtung 1 mit lediglich einem Leuchtenkopf 12. Erkennbar ist die im Wesentlichen U-förmige Bauform des Trägersystems 30 sowie die erste Schwenkachse 31, um die das Steuerungsmodul 20 schwenkbar gelagert ist. Wie bereits voranstehend beschrieben, weist die einem Patienten zugewandte Vorderseite 20a des Steuerungsmoduls 20 vier Aufnahmen 41-44 zur Aufnahme von modular ausgestalteten Leuchtenköpfen 11 - 14 (vgl. Figur 1) auf, wobei lediglich die zweitoberste Aufnahme 42 mit einem Leuchtenkopf 12 besetzt ist. Ebenfalls dargestellt sind Kontaktverbindungen 90 zum elektrischen und/oder kommunikationstechnischen Verbinden der Leuchtenköpfe mit dem Steuerungsmodul 20 zum Steuern und/oder Übertragen von Informationen. Aufgrund der bereits beschriebenen zentralen Anordnung der Steuerungen der Leuchtenköpfe in dem Steuerungsmodul 20, insbesondere der Vorschaltgeräte, kann das Gewicht der Leuchtenköpfe 11-14 wesentlich verringert werden. Es entstehen somit gut handhabbare Einheiten, die leicht an dem Steuerungsmodul 20 angeordnet werden können. Vorzugsweise kann ein Anordnen und ein Entfernen beziehungsweise Trennen der Leuchtenköpfe 11-14 von dem Steuerungsmodul 20 ohne Werkzeug vorgenommen werden, sodass ein äußerst flexibles System bereitgestellt wird, das je nach individuellem Bedarf für den jeweiligen Patienten kombinierbar und anpassbar ist. Um einen sicheren Betrieb zu gewährleisten, müssen die Leuchtenköpfe 11-14 sicher an dem Steuerungsmodul 20 befestigt werden, sodass diese nicht versehentlich gelöst werden können. Gleiches gilt für eine elektrische Verbindung der Leuchtenköpfe 11-14 mit dem Steuerungsmodul 20.

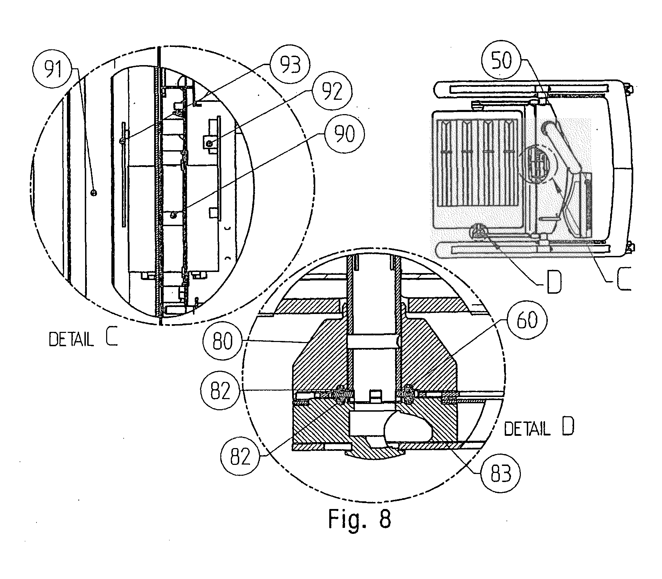

Wie bereits voranstehend angemerkt und in den Figuren 6-8 detailliert dargestellt, wird das Gehäuse 15 eines Leuchtenkopfes mittels seines Tragarmes 16 an dem Steuerungsmodul 20 befestigt, wobei der Tragarm 16 in einem linken Bereich L1 und einem rechten Bereich R1 mit jeweils einem Schwenkbolzen in eine als Kulisse ausgebildeten Abschnitt der Aufnahme 42 eingehängt wird. Aufgrund eines hierdurch gebildeten Drehpunktes 74 beschreibt der Tragarm 16 durch das Gewicht des Leuchtenkopfes 12 eine Drehbewegung nach unten und rastet mittels eines Rasthebels 75 selbstständig in eine untere Rastkulisse 71 des Steuerungsmoduls 20 ein, die einen zweiten Abschnitt der Aufnahme 42 darstellt. Dies verhindert ein versehentliches Lösen der Verbindung zwischen dem Leuchtenkopf 12 beziehungsweise seinem Tragarm 16 und dem Steuerungsmodul 20. Der Rasthebel 75fällt vorzugsweise durch sein Eigengewicht in seine Rastposition zurück. So braucht der Anwender den Leuchtenkopf 12 nicht durch einen separat vorzunehmenden Verriegelungsschritt selbst zu verriegeln. Vielmehr geschieht dies automatisch und kann somit nicht vergessen werden. Eine zum Einstecken der Kontaktverbindung 90 zwischen dem Tragarm 16 und dem Steuerungsmodul 20 notwendige Kraft wird ebenfalls durch das Eigengewicht des Leuchtenkopfes 12 bereitgestellt und somit eine entsprechende Kontaktierung automatisch hergestellt. Vorzugsweise ist der Tragarm 16 als U-förmiges Bauteil ausgebildet, wobei der Leuchtenkopf 12 zwischen den beiden U-Schenkeln angeordnet ist. Der Tragarm 16 kann demnach mit seinem die beiden Schenkel verbindenden Grundabschnitt mit dem Steuerungsmodul 20 verbunden werden. Ebenfalls vorzugsweise wird die Kontaktverbindung 90 mittig in diesem Grundabschnitt des Tragarms verbaut und mit einer Abdeckung 91 geschützt, sodass ein Berühren der Kontaktverbindung 90 verhindert wird (vgl. Figur 8, Detail C). Für diese Anordnung ist es jedoch notwendig, eine Steckerpaarung ohne eigene Verriegelung einzusetzen. Dies bedeutet, dass an der Kontaktverbindung 90 beim Entfernen des jeweiligen Leuchtenkopfes keine separate Verriegelung neben der Verrastung der Rasthebel zu lösen ist.

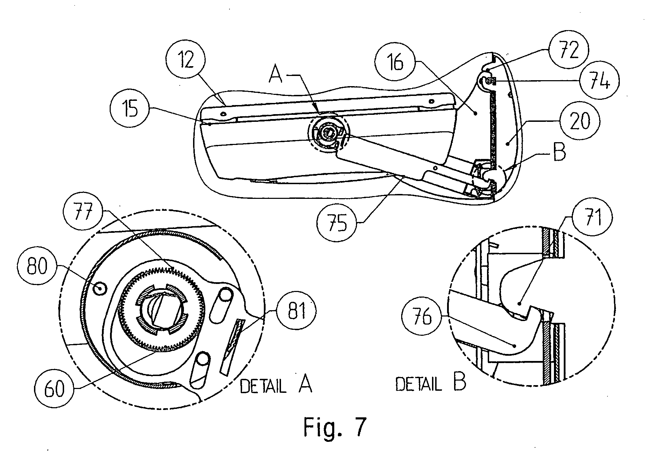

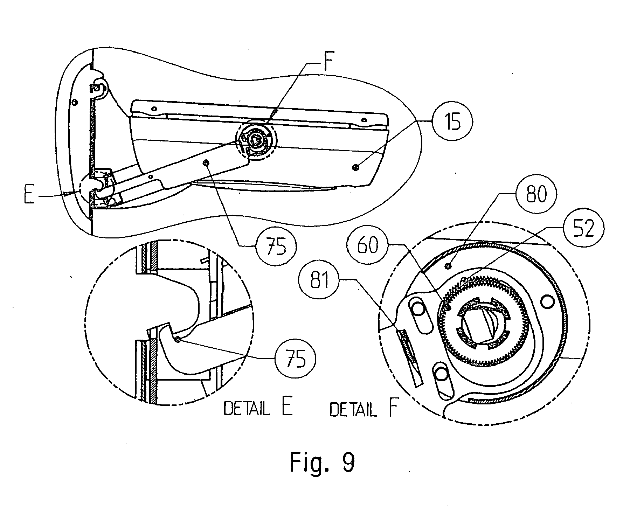

Wie in den Figuren 6-10 dargestellt, ist das Gehäuse 15 des Leuchtenkopfes 12 mittels einer Zahnscheibe 60 und einer Zahnkulisse 77 in einem oberen Teil des Rasthebels 75 gegen ein Verdrehen gesichert. In der dargestellten Ausführungsform steht lediglich der Rasthebel 75 auf der rechten Seite (Figur 7) in tatsächlichen Eingriff mit der Zahnkulisse 77, sodass eine Einhandbetätigung zum Verschwenken des Leuchtenkopfes 12 ermöglicht wird. Alternativ kann stattdessen der Rasthebel 75 der linken Seite in Eingriff stehen (nicht dargestellt) oder sogar beide, wobei in letztem Fall eine einhändige Bedienung nicht mehr möglich ist (beide letztgenannten Fälle nicht dargestellt). Um einen Neigungswinkel des Gehäuses 15 in der dargestellten Ausführungsform zu verstellen, wird der Rasthebel 75auf der rechten Seite R2 leicht nach oben gezogen. Die Zahnkulisse 77 gibt die Zahnscheibe 60 frei und das Gehäuse 15 kann mit der anderen Hand gedreht werden. Hierbei muss der Rasthebel 75 gehalten werden, da er durch sein Eigengewicht wieder in seine Ursprungslage zurückfällt und eine automatische Verrastung bewirkt. Um diese Bedienungsweise zu ermöglichen, ist der Rasthebel 75 auf der linken Seite L2 mittels einer asymmetrischen Fräsung 81 an einer Hülse 80 stets außer Eingriff gebracht. Dies bedeutet, dass die Zahnscheibe 60 nicht mit der Zahnkulisse 77 in Eingriff steht. Sie ist lediglich zur Verwendung von Gleichteilen vorgesehen, könnte jedoch auf weggelassen oder durch zahnlose Bauteile ersetzt werden. Eine Verrastung in der unteren Rastkulisse 71 an dem Schaltelement 20 ist hierbei gemäß Figur 9 dennoch beidseitig gegeben. Um die Verstellbewegung weich und gedämpft zu gestalten, können die Zahnscheiben 60 beidseitig mittels O-Ringen 82 gebremst werden. Über eine Verschraubung der Hülse 80 mit dem Lager 83 werden die O-Ringe 82 gegen die Zahnscheiben 60 gepresst.

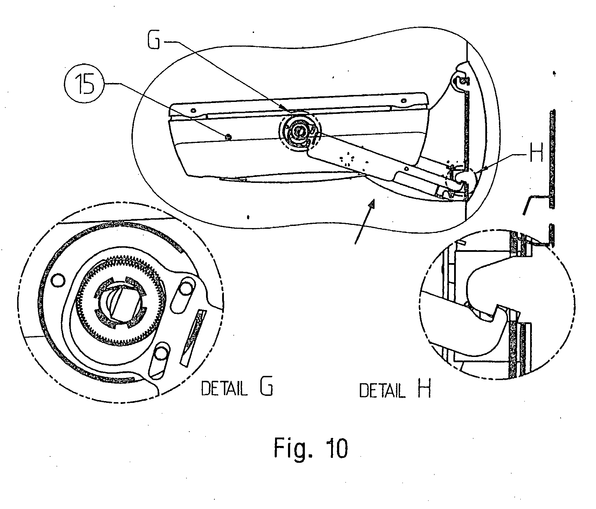

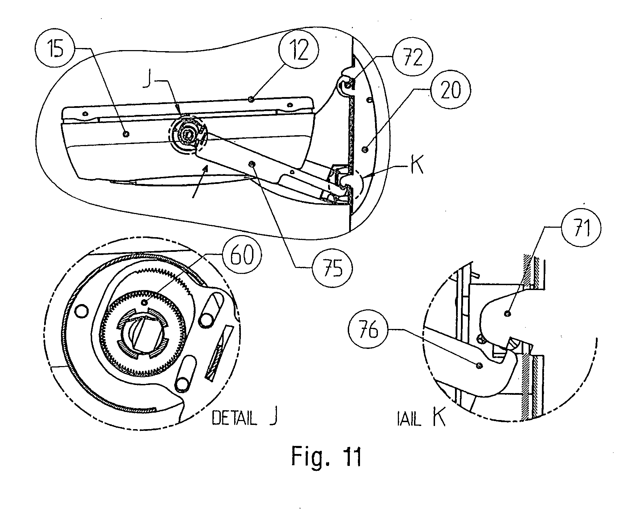

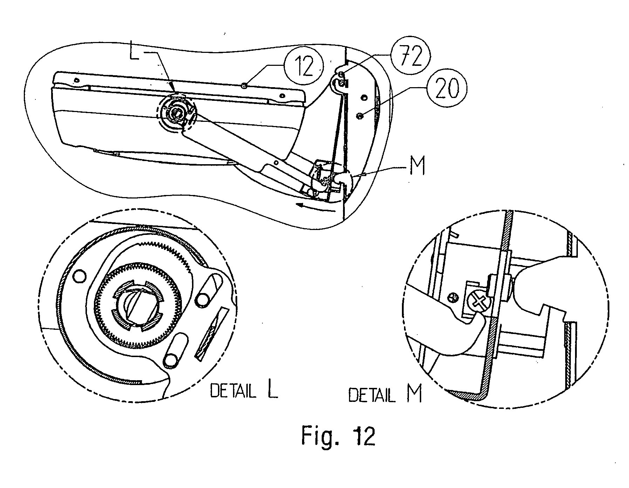

Die Figuren 11 und 12 zeigen den Leuchtenkopf 12 nach Figur 9 und 10 in einem entriegelten Zustand des Rasthebels 75 und der Zahnscheibe 60. Um den Leuchtenkopf 11-14 von dem Steuerungsmodul 20 zu trennen, werden beide Rasthebel 75 im Bereich des Gehäuses 15 vollständig nach oben gezogen. Rastnasen 76, die an einem entgegengesetzten Ende der Rasthebel 75 angeordnet sind, werden dabei aus der Rastkulisse 71 entfernt. Der Leuchtenkopf 12 kann anschließend in einer Schwenkbewegung von dem Steuerungsmodul 20 weg leicht angehoben werden und anschließend aus dem als Kulisse ausgebildeten Abschnitt der Aufnahme 72 genommen werden.

Aufgrund der dargestellten Möglichkeit einer freien Steckbarkeit und Kombinationsmöglichkeit der Leuchtenköpfe und deren unterschiedlichen Ausführungsformen und Bestückungen, insbesondere UV-A oder UV-B-Strahlungsquellen, müssen diese am jeweiligen Steckplatz mit der entsprechenden Bestückung erkannt werden. Hierzu erkennt das Steuerungsmodul 20 den jeweiligen Leuchtenkopf 11-14 sowie die gewählte Aufnahme. Jede Aufnahme verfügt über eine an dem Steuerungsmodul 20 vorgesehene Kodier-Platine 92, sodass eine eindeutige Kodierung zugeordnet werden kann. Dies geschieht beispielsweise mittels Jumpern, die entsprechend gesetzt werden können. Jeder Leuchtenkopf 11-14 ist darüber hinaus vorzugsweise mit einem Speichermedium 93 ausgestattet, auf dem die Spezifikationen des Leuchtenkopfes 11-14 doppelt gespeichert sind. Diese Spezifikationen umfassen beispielsweise Leuchtmittelbestückung, Intensität, Betriebsdauer des jeweiligen Leuchtenkopfes sowie dessen Seriennummer. So ist jeder Leuchtenkopf eindeutig identifizierbar. Die Informationen werden mittels einer Bus-Leitung in frei definierbaren Abständen oder beispielsweise beim Andocken an das Steuerungsmodul 20 übertragen.

### Bezugszeichenliste

- 1: Bestrahlungsvorrichtung

- 11: Leuchtenkopf
- 12: Leuchtenkopf
- 13: Leuchtenkopf
- 14: Leuchtenkopf
- 15: Gehäuse
- 16: Tragarm
- 17: zweite Schwenkachse

- 20: Steuerungsmodul
- 21: Lager
- 22: senkrechte Position
- 23: verschwenkte Position
- 24: Getriebe
- 25: Getriebe

- 30: Trägersystem
- 30a: Fuß
- 30b: Schenkel
- 30c: Strebe
- 31: erste Schwenkachse

- 41: Aufnahmen
- 42: Aufnahmen
- 43: Aufnahmen
- 44: Aufnahmen

- 50: Bedienelement
- 51: Schwenkarm
- 52: Bedienfeld Schwenkachse
- 53: dritte Schwenkachse

- 60: Zahnscheibe

- 71: untere Rastkulisse
- 72: untere Aufnahme
- 74: Drehpunkt
- 75: Rasthebel
- 76: Rastnase
- 77: Rastkulisse

- 80: Hülse
- 81: Fräsung
- 82: O-Ringe
- 83: Lager

- 90: Kontaktverbindung
- 91: Abdeckung
- 92: Kodier-Platine
- 93: Speichermedium

## Patentansprüche

1. Bestrahlungsvorrichtung zum Bestrahlen von Körperteilen eines Patienten mit
- einem Trägersystem (30), mehreren an diesem angebrachten Leuchtenköpfen (11, 12, 13,14) mit Lichtquellen für das Bestrahlen und
- einem Bedienelement (50) zum Bedienen der Bestrahlungsvorrichtung (11),
**gekennzeichnet, durch** ein mit dem Trägersystem (30) verbundenes, als Schaltschrank oder Rack ausgebildetes, separates Steuerungsmodul (20) mit einer Steuerung zum Steuern der Leuchtenköpfe (11, 12, 13, 14), wobei die Leuchtenköpfe (11, 12, 13, 14) modular ausgeführt sind und Gehäuse (15) aufweisen, die mittels Tragarmen (16) lösbar mit dem Steuerungsmodul (20) verbunden sind und das Steuerungsmodul (20) um eine erste Schwenkachse (31) schwenkbar mit dem Trägersystem (30) verbunden ist und die Gehäuse (15) der Leuchtenköpfe (11, 12, 13, 14) relativ zu dem Steuerungsmodul (20) um jeweils eine zweite Schwenkachse (17) schwenkbar sind.

2. Bestrahlungsvorrichtung nach Anspruch 1, bei dem die erste Schwenkachse (31) und die jeweils zweite Schwenkachse (17) im Wesentlichen parallel zueinander ausgerichtet sind.

3. Bestrahlungsvorrichtung nach Anspruch 1 oder 2, bei dem der mindestens eine Leuchtenkopf (11,12,13,14) kommunikationstechnisch zum Austausch von Informationenen mit dem separaten Steuerungsmodul (20) verbunden ist.

4. Bestrahlungsvorrichtung nach Anspruch1, 2 oder 3, bei dem die erste Schwenkachse (31) im Wesentlichen durch den Schwerpunkt des Steuerungsmoduls (20) verläuft.

5. Bestrahlungsvorrichtung nach einem der Ansprüche 1 bis 4, bei dem das Trägersystem (30) fahrbar zum Verfahren der Bestrahlungsvorrichtung (1) ausgebildet ist.

6. Bestrahlungsvorrichtung nach einem der Ansprüche 1 bis 5, bei dem das Bedienelement (50) schwenkbar um mindestens eine dritte Schwenkachse (53) mit dem Steuerüngsmodul (20) verbunden ist.

7. Bestrahlungsvorrichtung nach einem der Ansprüche 1 bis 6, bei dem der mindestens eine Leuchtenkopf (11,12,13, 14) jeweils mittels lösbarer Steckverbindungen mit dem Steuerungsmodul (20) verbunden ist.

8. Bestrahlungsvorrichtung nach einem der Ansprüche 1 bis 7, bei dem der mindestens eine Leuchtenkopf (11,12,13, 14) um mindestens 200° um dessen zweite Schenkachse (17) schwenkbar ist.

9. Bestrahlungsvorrichtung nach einem der Ansprüche 1 bis 8, bei dem der mindestens eine Leuchtenkopf (11,12,13, 14) jeweils mindestens einen Tragarm (16) zum lösbaren Verbinden mit mindestens einem an dem Steuerungsmodul (20) ausgebildeten Aufnahmeelement (41,42,43,44) zur mindestens teilweisen Aufnahme des mindestens einen Tragarms (16) umfasst.

10. Bestrahlungsvorrichtung nach Anspruch 9, bei dem der mindestens eine Tragarm mindestens einen Rasthebel zum wahlweisen Verrasten des Leuchtenkopfs (11,12,13,14) an dem Steuerungsmodul (20) umfasst.

11. Bestrahlungsvorrichtung nach einem der Ansprüche 1 bis 10, bei dem der mindestens eine Leuchtenkopf (11,12, 13,14) mittels einer Rastanordnung (77,60) relativ zu dem Steuerungsmodul (20) wahlweise fixierbar ist.

## Claims

1. Irradiation device for irradiating body parts of a patient comprising
- a carrier system (30), several light heads (11, 12, 13, 14) attached to this having light sources to produce the irradiation and
- a control element (50) to control the irradiation device (11),
**characterized by** a separate control module (20) connected with the carrier system (30) and formed as switchgear cabinet or rack, having a control for controlling the light heads (11, 12, 13, 14), wherein the light heads (11, 12, 13, 14) are modularly implemented and have housings (15), which are detachably connected with the control module (20) by means of supporting arms (16), the control module (20) is pivotably connected about a first pivot axis (31) with the carrier system (30) and the housings (15) of the light heads (11, 12, 13, 14) can be pivoted relative to the control module (20) about a respective second pivot axis (17).

2. Irradiation device according to claim 1, wherein the first pivot axis (31) and the respective second pivot axis (17) are aligned essentially parallel to each other.

3. Irradiation device according to claim 1 or 2, wherein the at least one light head (11, 12, 13, 14) is connected communicatively for exchange of information with the separate control module (20).

4. Irradiation device according to claim 1, 2 or 3, wherein the first pivot axis (31) extends essentially through the centre of gravity of the control module (20).

5. Irradiation device according to any one of claims 1 to 4, wherein the carrier system (30) is formed movably for moving the irradiation device (1).

6. Irradiation device according to any one of claims 1 to 5, wherein the control element (50) is connected pivotably with the control module (20) about at least a third pivot axis (53)

7. Irradiation device according to any one of claims 1 to 6, wherein the at least one light head (11, 12, 13, 14) is connected with the control module (20) by detachable plug and socket devices in each case.

8. Irradiation device according to any one of claims 1 to 7, wherein at least one light head (11, 12, 13, 14) can be pivoted by at least 200° about its second pivot axis (17).

9. Irradiation device according to any one of claims I to 8, wherein the at least one light head (11, 12, 13, 14) respectively includes at least one supporting arm (16) operative for detachable connection with at least one holder element (41, 42, 43, 44) formed on the control module (20) for at least a partial holding of the at least one supporting arm (16).

10. Irradiation device according to claim 9, wherein the at least one supporting arm includes at least one locking lever operative for optional engagement of the light head (11, 12, 13, 14) on the control module (20).

11. Irradiation device according to any one of claims 1 to 10, wherein the at least one light head (11, 12, 13, 14) is optionally fixable by means of a detent arrangement (77, 60) relative to the control module (20).

## Revendications

1. Dispositif d'irradiation de parties du corps d'un patient comportant :
- un système support (30), plusieurs têtes lumineuses (11, 12, 13, 14) montées sur ce système et munies de sources lumineuses permettant l'irradiation, et
- un élément d'actionnement (50) permettant d'actionner le dispositif d'irradiation (11),
**caractérisé par**
un module de commande (20) indépendant relié au système-support (30), réalisé sous la forme d'une armoire de distribution ou d'un rack, et comprenant un système de commande pour permettre de commander les têtes lumineuses (11, 12, 13, 14), ces têtes lumineuses (11, 12, 13, 14) étant modulaires et comprenant des boîtiers (15) qui sont reliés de manière amovible avec le module de commande, au moyen de bras support (16), le module de commande (20) étant relié au système support (30) en pouvant pivoter autour d'un premier axe de pivotement (31), et les boîtiers (15) des têtes lumineuses (11, 12, 13, 14) étant respectivement mobiles en pivotement relativement au module de commande (20) autour d'un second axe de pivotement (17) respectif.

2. Dispositif d'irradiation conforme à la revendication 1, dans lequel le premier axe de pivotement (31) et les seconds axes de pivotement respectifs (17) sont orientés essentiellement parallèlement.

3. Appareil d'irradiation conforme à la revendication 1 ou 2 dans lequel la tête lumineuse (11, 12, 13, 14) est reliée par des technologies de communication avec le module de commande indépendant (20) pour permettre l'échange d'informations.

4. Dispositif d'irradiation conforme à la revendication 1, 2 ou 3 dans lequel le premier axe de pivotement (31) passe essentiellement par le centre de gravité du module de commande (20).

5. Dispositif d'irradiation conforme à l'une des revendications 1 à4, dans lequel le système-support (30) est transportable pour permettre de déplacer le dispositif d'irradiation (1).

6. Dispositif d'irradiation conforme à l'une des revendications 1 à 5, dans lequel l'élément d'actionnement (50) est relié avec le module de commande (20) en pouvant pivoter autour d'au moins un troisième axe de pivotement (53).

7. Dispositif d'irradiation conforme à l'une des revendications 1 à 6, dans lequel la tête lumineuse (11, 12, 13, 14) est respectivement reliée au module de commande (20) par des liaisons à fiches amovibles.

8. Dispositif d'irradiation conforme à l'une des revendications 1 à 7, dans lequel la tête lumineuse (11, 12, 13, 14) peut pivoter d'au moins 200° autour de son second axe de pivotement (17).

9. Dispositif d'irradiation conforme à l'une des revendications 1 à 8, dans lequel la tête lumineuse (11, 12, 13, 14) comprend respectivement au moins un bras-support (16) pour permettre sa liaison amovible avec au moins un élément de réception (41, 42, 43, 44) formé sur le module de commande (20) pour permettre la réception au moins partielle de ce bras-support (16).

10. Dispositif d'irradiation conforme à la revendication 9, dans lequel le bras-support comprend au moins un levier d'encliquetage pour permettre l'encliquetage sélectif de la tête lumineuse (11, 12, 13, 14) sur le module de commande (20).

11. Dispositif d'irradiation conforme à l'une des revendications 1 à 10, dans lequel la tête lumineuse (11, 12, 13, 14) peut être sélectivement fixée relativement au module de commande (20) par un dispositif d'encliquetage (77, 60).
